# EUROPEAN PATENT APPLICATION

(11) **EP 1 815 821 A1**
(43) Date of publication of application: **08.08.2007**
(21) Application number: 06002107.8
(22) Date of filing: 02.02.2006
(51) Int. Cl.: A61F 2/06, A61M 25/10, A61M 25/02, A61M 16/04

(54) **Stent delivery system**

(71) Applicant: Dr. Karel Volenec - ELLA - CS, 500 06 Hradec Kralove (CZ)
(72) Inventor: Jan, Danis, 2410 Hainburg an der Donau (AT); Karel, Volenec, 500 06 Hradec Kralove (CZ)

(57) **Abstract**

The stent delivery system is used for introducing and deploying a self-expanding stent (10) into the esophagus.
It consists of an expandable member - typically, but not limited to, an inflatable balloon (7) in the distal portion thereof. The inflatable balloon (7), when inflated in the stomach, leans against the cardia, which prevents the stent delivery system to be removed through the cardia and esophagus so as to allow accurately deploy the self-expanding stent (10). Once deployed, the inflatable balloon (7) is contracted and the stent delivery system is removed.

## Description

This invention relates generally to medical devices. More particularly, the field of art into which this invention falls is devices/tools used for introducing and deploying stents, more specifically the self-expanding stents to be implanted into the esophagus.

Stents to be implanted into the esophagus are nowadays commonly introduced and deployed by using devices most often equipped with a non-traumatic soft tip at its distal end (i.e. the end more distant from a physician). The non-traumatic soft tip is fastened to the very end of a pusher, which is coaxially connected with a sheath; the stent is loaded in a collapsed condition between the pusher and sheath in a distal portion thereof such that the distal portion of the sheath overlaps the whole length of the collapsed stent, tightly adhering to the soft tip. When the delivery system with the stent in a collapsed state is introduced to a desired place in the anatomy, a physician starts to pull the sheath proximally (i.e. towards him/herself), which releases the collapsed stent from its distal end; the stent radially expands.

It is very difficult to accurately deploy self-expanding stents because they get irregularly shorten and/or a position of the distal portion of the delivery system with the collapsed stent in the esophagus can only be guided either endoscopically or fluoroscopically, which may be worsen with e.g. esophageal bleeding and the like; this may lead to a stent misplacement.

The submitted invention of the delivery system allows an accurate positioning of the stent in the esophagus and excludes its misplacement.

The object of the present invention of the stent delivery system is to allow an accurate positioning of stents in the esophagus. It has been achieved by using an expandable member - typically, but not limited to, an inflatable balloon, located in the distal portion of the pusher.

The delivery system with the collapsed stent is introduced into the esophagus such that the distal portion thereof reaches the stomach. Then the expandable member (e.g. balloon) is expanded by using typically the air, but not limited to, such that its diameter is larger than that of the esophagus, which makes the expandable member impossible to get back into the esophagus. Upon expanding the expandable member, the physician gently pulls the delivery system until the expandable member in the expanded state leans against the cardia - a sphincter between the stomach and esophagus. Then the delivery system is secured against movement and the stent may be released to expand. Once the stent is expanded, the expandable member is contracted to the original diameter and the whole delivery system is removed through the esophagus from the anatomy.
Fig. 1 is a side view of the preferred embodiment of the invention with the loaded stent in a collapsed state before use.
Fig. 2 is a side view of the preferred embodiment of the invention of Fig. 1 when the sheath is partly pulled proximally and the expandable member - balloon - is in the expanded state.
Fig. 3 is a side view of the preferred embodiment of the invention of Fig. 1 when the sheath is completely pulled proximally, the stent is released in the expanded state and the expandable balloon is contracted; the delivery system is ready to be removed from the anatomy.

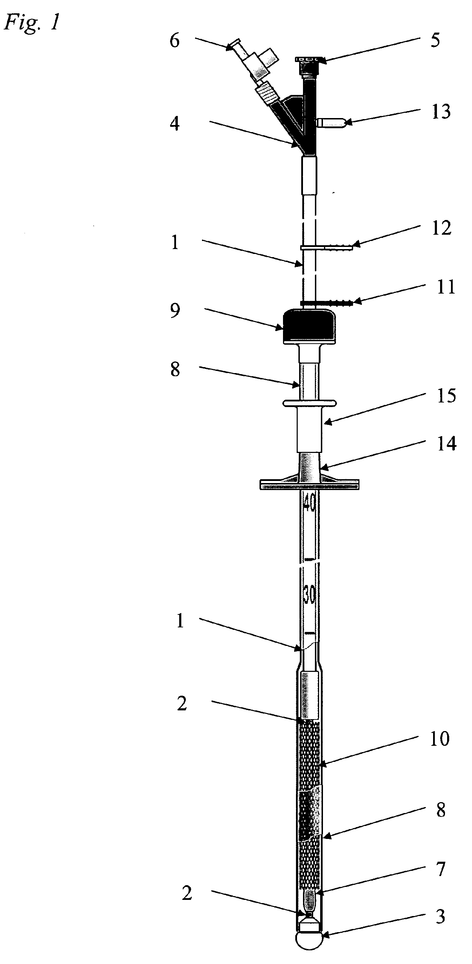

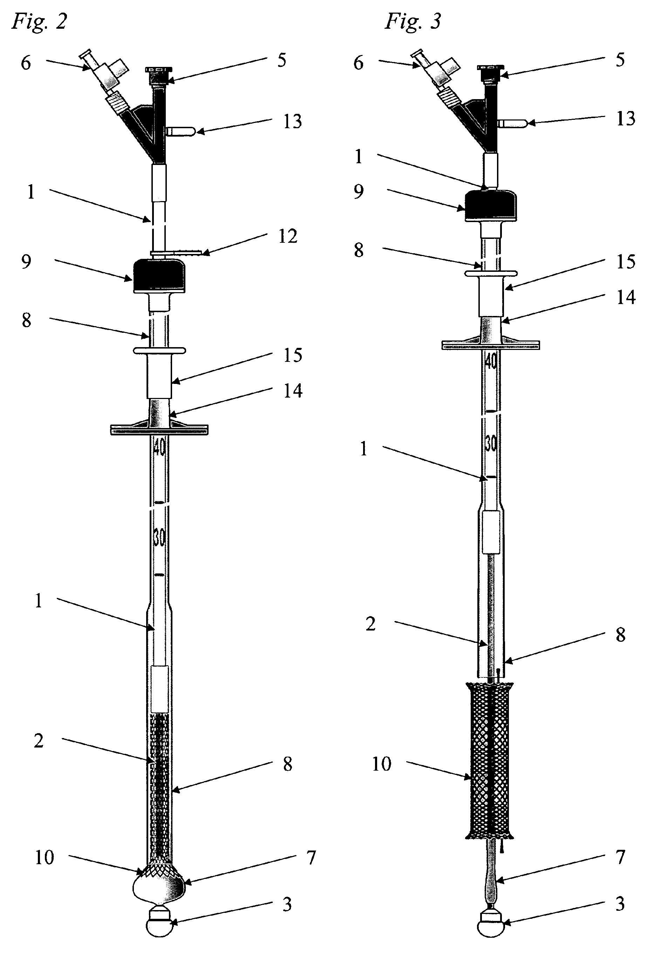

The stent delivery system of the present invention consists of a scaled pusher 1 to which a shaft 2 ended with non-traumatic tip 3 is fastened at its distal end; the scale helps a physician in positioning of the stent delivery system in a patient's anatomy. At the proximal end the pusher 1 is ended with a connector 4 having two ports with two separated lumens - a guide wire port 5 and syringe port 6. The guide wire port 5 serves for insertion of a guide wire (if a physician considers using a guide wire) that passes through the guide wire lumen of the pusher 1, shaft 2 and non-traumatic tip 3 out of the distal end of the stent delivery system. To the syringe port 6 typically, but not limited to, a syringe can be fastened; the syringe (or a similar device) serves for inflation of an inflatable balloon 7, a proximal end and distal end of which are circumferentially fastened to distal portion of the shaft 2. By using the syringe, the air (or other suitable medium) is pumped up into the syringe port 6, through the inflation lumen of the pusher 1 and shaft 2, and further through a perforation of the shaft 2 into the inflatable balloon 7, which makes it inflated; a backflow of the air is impossible due to the syringe port 6, which is equipped with a one-way valve. To avoid an inflatable balloon rupture due to an excessive pressure, the connector 4 is equipped with a safety balloon 13, which gets inflated if the syringe exerts an excessive pressure. A sheath 8 with a handle 9 is coaxially put on the pusher 1 and shaft 2 such that the distal end of the sheath 8 tightly adheres to the non-traumatic tip 3. A stent 10 in a collapsed state is longitudinally loaded between the sheath 8 and the shaft 2 such that its proximal end is located by the distal end of the pusher 1 and distal end overlaps one third of the inflatable balloon 7 (as shown in Fig. 1). The sheath 8 is axially movable in a proximal direction. To avoid an unwanted movement, two clips are also put on the pusher 1. A clip A 11 is put tightly to the sheath handle 9; if it is removed, the sheath 8 can be pulled to a clip B 12, which uncovers the inflatable balloon 7 and a distal end of the stent 10 (as shown in Fig. 2). If the clip B 12 is removed as well, the sheath 8 can be pulled further to the connector 4, which uncovers the stent 10. The sheath 8 is equipped with a block 14 and its brake 15; both parts are located distally to the sheath handle 9, adhering thereto; they are slidable in a distal direction. The block 14 with brake 15 secures a position of the introduced stent delivery system from a proximal direction.

The distal end of the stent delivery system with loaded stent 10 is introduced through a patient's mouth, esophagus and cardia into a stomach until the block 14 touches the patient's mouth. The clip A 11 is removed and the scaled pusher 1 is pushed distally, which pushes the inflatable balloon 7 out of the sheath 8, until the clip B 12 reaches the sheath handle 9. The syringe is fastened to the syringe port 6; an appropriate volume of the air is pumped up thereinto, which inflates the inflatable balloon 7; the stent delivery system is subsequently pulled proximally until a resistance is felt - i.e. the inflatable balloon 7 leans against the cardia. The block 14 is then slid distally, tightly to a patient's mouth; subsequently the brake 15 is slid distally and pressed onto the block 14, which prevents the block 14 from movement. Now the stent delivery system is fixed between the stomach and mouth, which allows the stent 10 to be deployed accurately. The clip B 12 is now removed and the sheath 8 is then pulled proximally, which uncovers the stent 10; it, being uncovered from its distal end, radially expands (as shown in Fig. 3). When the stent is deployed, the syringe port 6 can be loosened by unscrewing, which allows a backflow of the air and the inflatable balloon 7 deflates. Once it is contracted, the stent delivery system can be safely removed.

## Claims

1. A stent delivery system designed for an accurate deployment of a stent in the esophagus **characterized in that** it comprises an expandable member located in the distal portion thereof such that the stent delivery system is introduced through the patient's mouth, esophagus and cardia such that the distal portion of the stent delivery system reaches the stomach wherein the expandable member is expanded by means of an appropriate medium or mechanism such that the expandable member in an expanded state cannot get back through the cardia and esophagus, which allows the stent delivery system to be pulled back only until the expandable member in an expanded state leans against the cardia, which secures a position of the stent delivery system.

2. The stent delivery system according to claim 1, **characterized in that** the expandable member is an inflatable balloon (7) of which a proximal and distal ends are circumferentially fastened to a shaft (2); said inflatable balloon (7) is inflated with the air, by using a syringe, that is pumped up into a syringe port (6), being located in the proximal portion of the stent delivery system, and further through an inflation lumen of a pusher (1) and shaft (2).

3. The stent delivery system according to claim 2, **characterized in that** the syringe port (6) is equipped with a one-way valve making a backflow of the pumped-up air impossible.

4. The stent delivery system according to claim 2, **characterized in that** the inflation method is equipped with a safety balloon (13), being located out of a patient's body, which gets inflated if the syringe exerts an excessive pressure, which avoids a rupture of the inflatable balloon (7).

5. The stent delivery system according to claim 1, **characterized in that** a sheath (8) with a handle (9) is coaxially put on the pusher (1) and shaft (2) such that the distal end of the sheath (8) tightly adheres to a non-traumatic tip (3), being located at the very distal end of the stent delivery system, such that the sheath (8) is axially movable in a proximal direction, which is, however, prevented by a clip A (11) and a clip B (12) located in the proximal portion of the pusher (1) such that if the clip A (11) is removed, the sheath (8) can be pulled proximally to uncover the inflatable balloon (7) and if the clip B (12) is removed, the sheath (8) can be pulled further proximally to uncover the stent (10).

6. The stent delivery system according to claim 5, **characterized in that** the sheath (8) is equipped with a block (14) and its brake (15) located distally to the sheath handle (9) adhering thereto; said block (14) is slid distally, tightly to a patient's mouth and said brake (15) is slid distally and pressed onto the block (14) to prevent it from movement to secure the position of the stent delivery system.

7. The stent delivery system according to claim 1, **characterized in that** the stent (10) in a collapsed state is longitudinally loaded between the sheath (8) and shaft (2) such that its proximal end is located by the distal end of the pusher (1) and distal end overlaps one third of the inflatable balloon (7).
